# EUROPEAN PATENT SPECIFICATION

(11) **EP 2 210 637 B1**
(45) Date of publication and mention of the grant of the patent: **12.10.2016**
(21) Application number: 08829174.5
(22) Date of filing: 14.08.2008
(51) Int. Cl.: A61M 11/06, A61M 11/04, A61M 15/08

(54) **PORTABLE ESSENCE VAPORISER**
TRAGBARER ESSENZ-VAPORISATOR
VAPORISATEUR D'ESSENCES PORTATIF

(30) Priority: 28.08.2007 ES 200702406
(43) Date of publication of application: 28.07.2010
(73) Proprietor: Fernández Pernía, Jorge, 09006 Burgos (ES)
(72) Inventor: Fernández Pernía, Jorge, 09006 Burgos (ES)
(74) Representative: ZBM Patents - Zea, Barlocci & Markvardsen
(86) International application number: PCT/ES2008/070167
(87) International publication number: WO 2009/030800

(56) References cited:
- EP-A1- 0 858 744
- EP-A1- 1 736 065
- WO-A1-99/11311
- WO-A1-99/11311
- WO-A1-2006/097668
- WO-A2-2006/009923
- US-A- 4 141 369
- US-A- 4 360 018
- US-A- 4 825 863
- US-A- 4 825 863
- US-A- 5 067 499
- US-A- 5 322 057
- US-A1- 2003 217 750
- US-A1- 2003 217 750
- US-A1- 2007 003 751

## Description

The present invention relates to a portable essence vaporizer that extracts an essence and supplies it to a user, comprising a heat source for heating an air flow which is passed through a substance from which the essence is to be extracted, and then is supplied to the user.

### PRIOR ART

In the state of the art and in the market, there are several known electrical vaporizing devices for substances such as tobacco, aromatic or medicinal plants, etc. These devices cause the selective evaporation of essences, flavors, aromas or other active ingredients contained in the previously mentioned substances. The vapor of the essences is extracted by heating the substance in its solid or liquid state to a temperature equal or higher than the evaporation point of the essence that is contained in said substance, causing it to change to a gaseous state and thereby turning into vaporized essence.

There are several methods used to apply heat to a substance in order to cause evaporation. The two main ones are: extraction with heating by conduction or extraction with heating by convection.

The method of heating with thermal conduction, such as the one used in the patent US 2003/0150451, involves applying heat to a surrounding surface that encircles the substance until the evaporation temperature of the essence is reached. One of its disadvantages is that the system has a big thermal inertia, so that when one wants to stop the evaporation, the surrounding surface has to be cooled down with its corresponding energy losses. Or alternatively, the substance has to be removed from the surrounding surface, making it burdensome for the user who has to do this operation repeatedly. Another inconvenience of this method is that the quantity of vapor extraction is independent of the flow inhaled by the user. This means that the user cannot control the amount of vapor extracted. The device evaporates the same quantity independently of whether there has been inhalation, not taking into account whether the user actually uses the vapor or not.

Vaporizers that use methods of extraction by thermal convection are based on the heating of air or other gas until a temperature equal or higher than the evaporation point of the essence is reached. The method of extraction by convection has been proven to give better results time wise than the methods of extraction by conduction since the amount of obtained vapor is directly proportional to the warm air flow going through the substance. This way, if the warm air does not pass through the substance, there is no evaporation. The advantage is that the user has control over the quantity of extracted vapor since it is proportional to the volume of warm air that passes over the substance during the inhalation process.

The advantages shown in the method of extraction by air or gas convection compared to the heating method by conduction make its use more recommendable.

A vaporizer known in the prior art is described in the patent with publication number US4141369, relating to an essence extracting vaporizer by means of forced thermal convection (heating of air or other gas until a temperature equal or higher than the essence's point of evaporation is reached). Said vaporizer uses a heat source to increase the temperature of the air which, when passing through the substance, causes the evaporation of its essence. And, in case of a substance made of combustible material, prevents its combustion with this same method; and it furthermore includes an air chamber to isolate the heat source from the casing of the vaporizer.

However, the air inside this known vaporizer is unable to reach high temperatures, especially if it has to be adapted to a portable size. This is due to the limited amount of heat that can be transferred to air, and to the huge dimensions the air chamber should have to avoid an excessive exterior temperature.

For this reason, this known device has a low performance and a poor extraction efficiency.

The purpose of the current invention is to provide a compact portable essence vaporizer of small dimensions in which air can reach high temperatures for a satisfactory extraction as well as having a good energy performance, and as a result, a reduced electricity consumption.

From this point on, the term substance will be used when to refer to the chemical compounds, both in the form of plant or solid or liquid extract, from which one wants to obtain essence or essence vapor (which is the term that will be used hereinafter for the product obtained through the extraction process).

WO2006/009923A2 discloses an apparatus for vaporizing medical herbs and essences that uses an electrical power driven medium to vaporize the herbs and essences, having a heating element assembly within a shield.

EP0858744A1 discloses a flavor generation piece that incorporates a body of a material containing a flavor substance, and a heater to heat the piece.

### BRIEF DESCRIPTION OF THE INVENTION

The vaporizer of the current invention extracts the essence of a substance placed in the vaporizer, through a heating method using thermal convection of air or another gas. The vaporizer can be used as an aroma or taste extractor for spices in the cooking process; as an extractor of odor in aromatherapy treatments for psychosomatic conditions; for the extraction of active ingredients and their medical use; as a vaporizer and inhaler of nicotine for tobacco users, reducing the risks which come with the consumption through combustion.

The current invention relates to a portable essence vaporizer that extracts an essence and supplies it to a user as claimed in claim 1.

The essence extraction can be performed at higher air temperatures than in other known devices, with a better energy performance and with reduced dimensions that make the device easy to handle, easy to transport, etc.

In some embodiments the nanoporous insulating material is microporous, with a pore size smaller than 2 nm.

The insulation can include monolithic structures of metal oxides, amorphous silica and glass filaments. In the context of the current invention, the term "glass filaments" refers to filaments, of any material, which are crystallized in an amorphous or vitreous manner.

The structure and components give the material a very low thermal conductivity, even at high temperatures, allowing for a reduction of the thickness of the insulation and consequently of the size of the vaporizer, as well as contributing to a very low contraction when cooling down. Additionally, they limit the external temperature of the vaporizer so that it can be gripped without risk of burns or degradation of the outer casing. Furthermore, they reduce the energy losses so as to make the system perform with optimal electricity consumption.

The heat exchanger comprises an open cell foam structure. Thanks to the high specific surface area of these materials, the exchanger conducts heat better and faster to the air surrounding it, improving the performance of the vaporizer. Consequently, the user can extract a high quantity of essence vapor in less time and with less inhaled volume.

In some embodiments, the open cell foam structure has a specific surface area of between 50 and 200 cm²/cm³.

The open cell foam structure can be metallic or ceramic. The ceramic foam structure provides a higher specific heat capacity than metal, whereas the metallic structure offers better thermal conductivity from the heat source to the heat exchanger.

In embodiments of the invention, the vaporizer comprises a duct for air flow circulation, said duct including an extraction chamber arranged downstream from the heat source and used for the purpose of housing the substance.

In one embodiment, the vaporizer also comprises a humidifier placed in such a way as to allow the air flow to go through it.

During the essence extraction process, such humidifier causes the warm and dry air that circulates from the exchanger to the exit of the vaporizer to absorb enough humidity to make the inhalation process more pleasant for the user in the case of essence extraction through suction.

The humidifier can be placed downstream from the extraction chamber so that the air flow goes through it after the essence extraction. Alternatively, the humidifier can be place upward of the extraction chamber, so that the air flow goes through it before the essence extraction.

Preferably, the vaporizer includes an inhalation nozzle through which the user can inhale to cause the air flow going through the substance from which the essence is to be extracted and subsequently being inhaled by the user.

According to an advantageous embodiment, the vaporizer furthermore comprises a vapor collector whose purpose it is to filter the exhaled vapor by the user after performing an essence extraction.
This way, the waste air exhaled by the user who has inhaled the essence is filtered through the vapor collector and, through the activated carbon, filtering out the particles that are exhaled in the collector, thereby exhausting to the exterior nothing but clean filtered air.

### DESCRIPTION OF EMBODIMENTS OF THE INVENTION

An embodiment of the invention will be described next by way of non-limiting example, with reference to the attached drawings, in which:
Figure 1 shows a view of the cross-section of a first embodiment of the essence vaporizer according to the present invention; and
Figures 2 to 4 show other alternative embodiments of the essence vaporizer.

The invention provides an essence vaporizer for extracting the essence of a substance using the method of evaporation by displacement of hot air, known as forced thermal convection.

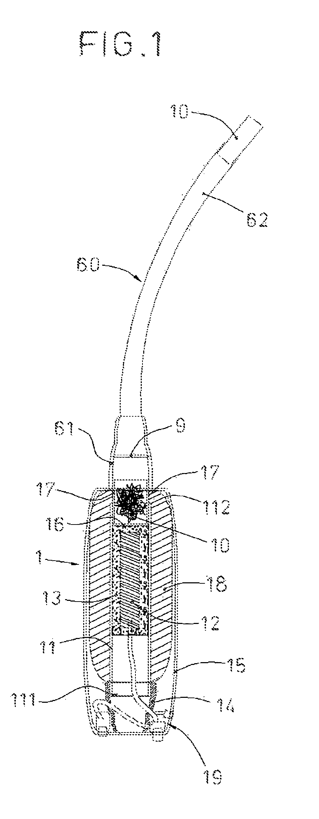

In the embodiment of figure 1, the vaporizer comprises a chamber (10) for containing in its interior a substance of which its essence is to be extracted; and a tubular element (11) that, at its center, contains the assembly of the heat source (12) and a heat exchanger (13), used to heat up the air in its interior and thus allowing for the essence extraction of the substance contained in the chamber (10) when the user inhales such air.

The tubular element (11) and the chamber (10) are surrounded by an insulation (18). The assembly is to be found inside the interior of the casing (15), which is open at its ends and is linked to the inhalation nozzle (60).

In order to extract the essence, the user inhales air through the nozzle (60) optionally with a mask (not shown), generating an air flow that enters the device at the lower end of the casing (15), and goes through the heat exchanger (13), the chamber (10), and the nozzle (60), thus reaching the user.

The casing (15) also works as an adequate grip so that the user can hold the vaporizer in his hands.

In the current embodiment, the thermal insulation (18) is made of a nanoporous material, and more specifically a microporous material, with monolithic structures of metal oxides, amorphous silica and glass filaments, pressed together to form a solid block. This gives the insulation a very low thermal conductivity and a good dimensional stability at high temperatures (with very little contraction in the cooling down process).

The nanoporous material has a pore size inferior to 50 nm., and preferably inferior to 2nm. (microporous material).

Microporous materials suitable for the insulation (18), as well as their properties and manufacturing processes, are described in the patent applications WO2005/040063 y WO2006/097668, which can be referred to for more information on this matter.

A specific example of a suitable insulation is the so-called MPS (R), from Microtherm (R).

The thermal insulation (18) is placed surrounding the tubular element (11) and in direct contact with it. Its thickness is so that the external surface area, in contact with the interior of the casing (15), maintains the temperature that allows for the object to be handled without risk of burns or degradation of the casing. The length of the thermal insulation covers at least the contact area between the tubular element (11) and the group formed by the heat source (12) and the heat exchanger (13),

As shown in figure 1, the tubular element (11) has a circular or polyhedral cross-section, open at its two ends, which constitute an air entrance point (111) and an air exit point (112). The materials used for the elements found within the tubular element (10) have to withstand the maximum working temperatures of the heat source.

The heat source (12) is a device consisting of electrodes that convert electrical energy into thermal energy. The electric current flow is proportional to the energy transformed into heat. By increasing or decreasing the electrical current with an electric controller (19) we can reach different exit temperatures for the air flow that goes through the exchanger (13). For instance, the heat source can be a FIREROD® cartridge heater manufactured by WATLOW®.

The heat exchanger (13) can be a stainless steel open cell foam heat exchanger, such as the one commercialized by AlCarbon, which has a high specific surface area.

Additionally, its tubular shape is such that the heat source (12) can be inserted in its interior to transmit energy throughout the whole surface area. Both the open cell foam structure and said tubular shape provide a large contact surface area with air that favors heat exchange. In another embodiment, the stainless steel foam could be substituted by a foam of a ceramic material, which has a higher specific heat capacity than steel.

At the air entrance point (111) of the tubular element (11), a tubular profile (14) which is open at its ends, is placed, in a sealing manner joining together the air entrance point (111) of the tubular element (11) and the air entrance of the casing. The power supply cables of the heat source (12) go through the tubular profile without compromising its sealing. The connecting tubular profile (14) could be made of silicone or some other elastomer that guarantees the sealing property and that can withstand the operational temperatures.

Within the tubular element (111), in contact with and facing one of the ends of the heat exchanger (13) neighboring the air exit (112), there is a mesh disc (16) with a mesh opening between 10 and 500 microns and with a cross-section that allows it to be inserted in the tubular element (11) adjusting itself to the internal diameter of the tubular element. In some of the embodiments, the mesh disc is made of stainless steel, preferably with a thread thickness of 200 microns and a mesh opening of 400 microns.

Next, a tubular element with a non-stick wall (17) is placed within the air exit (112) end of the tubular element (11), so that it supports the mesh disc (16) and defining in its interior, the chamber (10) for the substance of which the essence is to be extracted. This element (17) sticks out of the casing (15) in such a way that it makes it possible to mount the next component, in this case the nozzle (60).

The profile of the tubular element with non-stick wall (17) has a shape that allows it to stay firmly fitted pressing against the interior walls of the tubular element (11). In one of the embodiments the element is a circular profile manufactured of a polymer such as ECTFE (HALAR®).

The casing (15) is metallic, of tubular shape, and it contains in its interior the thermal insulation (18), the assembly of the tubular element (11), the heat source (12), the heat exchanger (13), and the mesh disc (16). Its air entrance and exit ends are open and remain in touch with the tubular profile (14) and the tubular element with non-stick wall (17) respectively.

The inhalation nozzle (60) is connected to the tubular element of non-stick wall (17). The entrance end (61) of the nozzle (60) can be inserted externally of the tubular element with non-stick wall (17) in such a way that they stay firmly fastened and allows for a good sealing to the extracted vapor. In other embodiments, the entrance end (61) can be inserted internally of the tubular element (17).

In the embodiment shown in figure 1, the nozzle (60) consists of a tubular element which changes cross-section from its vapor entrance end (61) to its vapor exit end (62). In its interior, it contains a mesh filter (9) of similar characteristics as the mesh disc (16). The filter (9) serves as a barrier to solid particles so that the substance or substance portions of which the essence is extracted do not enter the user's respiratory ways during inhalation, yet allowing the extracted vapor essence to do so.

The inhalation nozzle (60) can be made of an elastomer material such as for example silicone. The vapor exit end (62) can have a variable length to enhance user convenience and it has an inserted mouthpiece (10) with a pleasant feel to the user's mouth.

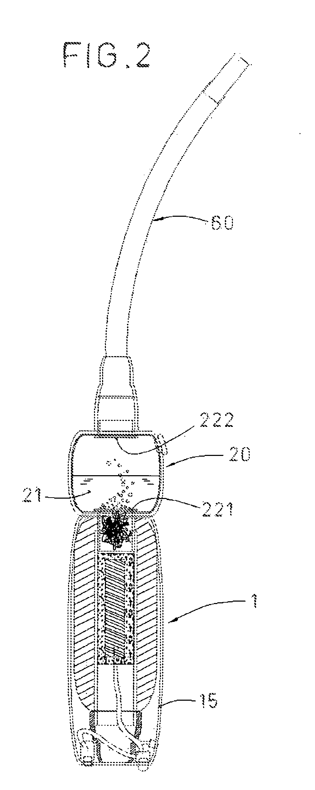

Figure 2 shows an embodiment in which the vaporizer includes furthermore an anti-spillage humidifier (20) that humidifies the vapor. It is placed between the evaporation chamber (10) and the inhalation nozzle (60).

This way, at the vapor exit of the vaporizer as a whole (1), which is the whole group contained within the casing (15) described up to now, an anti-spillage humidifier (20) is placed in series. The humidifier (20) comprises a water container (21) with an air entrance at its lower end, connected to the vapor exit of the vaporizer group (1). It also comprises an air exit in its upper end, connected to the nozzle (60). The entrance and exit of the humidifier (20) are equipped with hydrophobic membranes (221 and 222) that allow the vapor to pass but prevent water spillage from the container (21). The membranes can be substituted by non-return valves that perform the same anti-spillage function.

The air entrance of the humidifier (20) is connected in series to the vapor exit of the vaporizer group (1), as shown in figure 2, if one wishes to perform a dry extraction and a subsequent vapor humidification. However, the humidifier (20) can also be placed the other way around, i.e. connecting the air entrance of the vaporizer group (1) to the air exit of the humidifier (20) to establish an extraction with humid air.

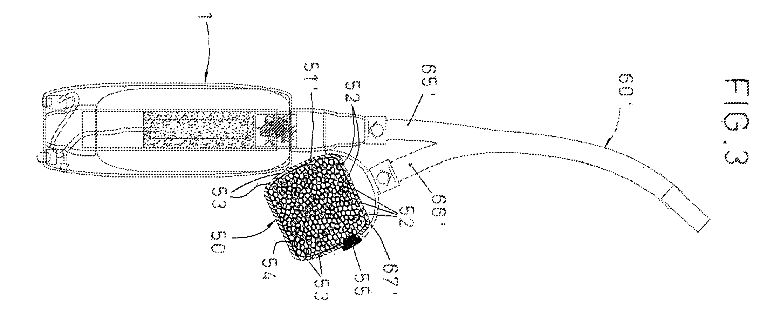

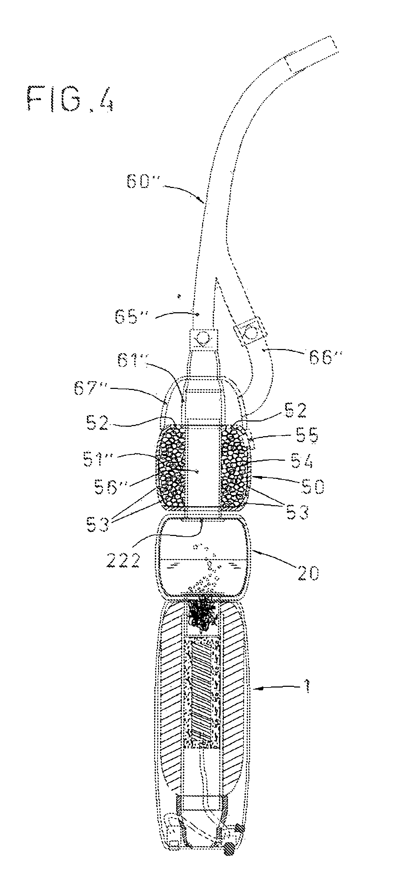

Figures 3 and 4 show variations of embodiments which furthermore comprise a vapor collector (50). The purpose of the vapor collector is to filter the air that the user expels into the air after inhaling the essence vapor for example in order to avoid smells.

In order to fit the vapor collector to this type of vaporizer, the inhalation nozzle has to have a different configuration than the nozzle (60) shown in figure 1, as will be explained next.

In figure 3, a vapor collector (50) comprises a carbon trap (51') and several air entrance points (52) and air exit points (53). The carbon trap (51') contains in its interior activated carbon particles (54). It is formed by a container with a mesh opening inferior to the minimum size of the carbon particles.

Also, the nozzle (60') in figure 2 comprises a first branch (65') coupled to the exit of the vaporizer as a whole (1) and a second branch (66') connected to a vapor collector (50). The two branches (65' and 66') of the nozzle (60') comprise non-return valves arranged in a suitable way so that the user can inhale the vapor essence through the first branch (65') and exhale the air through the second branch (66') all the way to the vapor collector (50). The branch (66') ends at an adaptor (67') suitable for being connected to the entrances (52) of the collector (50).

In the vapor collector (50), the vapor expelled by the user and ducted through the branch (65') flows from the entrances (52) all the way to the exits (53). Along the way it goes through the carbon particles (54), whose purpose is to trap any substance contained in the vapor.

The filling up and emptying of the activated carbon (54) from the interior of the collector (50) can be performed using a plug (55).

In figure 4, the vapor collector (50) is similar to that of figure 3 but it includes a central duct (56") so that it can be mounted downstream from the humidifier (20) in the direction of the vapor flow towards the user. As shown in the figure, this central duct (56") is placed between the membrane (222) of the humidifier (20) and the entrance (61 ") of the nozzle (60"), whereas the carbon trap (51") is configured of annular shape, provided around the duct (56").

As shown, the nozzle (60") comprises two branches (65" and 66") that include suitable non-return valves. The nozzle is connected to a vapor collector (50) in such a way that the user inhales the vapor with the essence through the first branch (65"), which is communication with the humidifier (20) through the central duct (56") of the vapor collector (50). The air exhaled by the user goes through the carbon filter of the collector (50).

Both branches (65" and 66") of the nozzle (60") end at an adaptor (67") that surrounds the lower far end (61 ") of the branch (65") and allows for a suitable fit between the branch (66') and the entrances (52) of the vapor collector.

The principle of operation of the embodiments in figures 3 and 4 is very similar. The main difference is the mounting system of the vapor collector (50), which in the case of figure 4 is better integrated in the device.

As it will be understood from the previous description, in practice the vaporizer comprises a series of modules (vaporizer group, humidifier, vapor collector) that can be connected in different ways, depending on the desired effect for each case. The device can be marketed with different modules and accessories (mouthpieces, adaptors, etc.) so that the user can choose at each moment the configuration that he or she desires.

Notwithstanding the fact that the invention has been described and shown with reference to one or more particular embodiments of the vaporizer, the person skilled in the art will be able to introduce changes and modifications, in accordance with circumstances, and substitute any element for any technical equivalent thereof, without departing from the scope of protection defined by the appended claims.

For instance, the skilled person will understand that although only certain particular embodiments of the vaporizer with humidifier and/or vapor collector have been described, due to the modular build-up of the device, other combinations and configurations are possible.

## Claims

1. Portable essence vaporizer for extracting an essence and supplying it to a user, which comprises a heat source (12) for heating up an air flow which is passed through a substance of which one can extract an essence, and is supplied to a user, further comprising a heat exchanger (13) provided in such a way that the heat generated by the heat source (12) is supplied to the air flow, and a thermal insulation (18) for insulating the heat source (12) and the heat exchanger (13) from the exterior, **characterized in that** the heat exchanger (13) comprises a tubular piece which comprises an open cell foam structure, the heat source (12) being provided at least partially inside the tubular piece, **in that** the thermal insulation (18) comprises a tubular element which at least partially surrounds the heat exchanger (13) and which comprises a nanoporous material with pores of a size of less than 50 nm, and **in that** the assembly is housed within a casing (15) which is intended to be gripped by a user.

2. Vaporiser according to claim 1, **characterized in that** the nanoporous material is microporous, with pores of a size of less than 2 nm.

3. Vaporizer according to any previous claim, **characterized in that** the insulation (18) comprises monolithic structures of metal oxides, amorphous silica and glass filaments.

4. Vaporizer according to any of claims 1 to 3, **characterized in that** the open cell foam structure has a specific surface area of between 50 and 200 cm²/cm³.

5. Vaporizer according to any of claims 1 to 4, **characterized in that** the open cell foam structure is metallic.

6. Vaporizer according to any previous claim, **characterized in that** it comprises a duct (14,11,17) for the circulation of the air flow, said duct comprising an extraction chamber (10) for housing the substance being provided downstream of the heat source (12).

7. Vaporizer according to any previous claim, **characterized in that** it furthermore comprises a humidifier (20) provided in such a way that the air flow passes through it.

8. Vaporizer according to claim 7, when it depends on claim 6, **characterized in that** the humidifier (20) is provided downstream of the extraction chamber (10) so that the air flow passes through it after extracting the essence.

9. Vaporizer according to claim 7, when it depends on claim 6, **characterized in that** the humidifier is provided upstream of the extraction chamber (10), so that the air flow passes through it before extracting the essence.

10. Vaporizer according to any previous claim, **characterized in that**, it comprises an inhalation nozzle (60; 60'; 60") through which a user inhales air so as to cause said air flow which passes through the substance of which the essence is to be extracted and which is inhaled by the user.

11. Vaporizer according to any previous claim, **characterized in that** it comprises a vapor collector (5) for filtering the vapor exhaled by the user after essence extraction.

## Patentansprüche

1. Tragbarer Essenzvaporisator zur Extraktion einer Essenz und zur Lieferung an einen Benutzer, welcher eine Wärmequelle (12) zur Erwärmung eines Luftstroms umfasst, welcher durch eine Substanz durchflossen wird, von welcher eine Essenz extrahiert werden kann, und an einen Benutzer geliefert wird, weiterhin umfassend einen Wärmeübertrager (13), der so bereitgestellt ist, dass die durch die Wärmequelle (12) erzeugte Wärme an den Luftstrom zugeführt wird, und eine thermische Isolierung (18) zum Isolieren der Wärmequelle (12) und des Wärmeübertragers (13) nach außen, **dadurch gekennzeichnet, dass** der Wärmeübertrager (13) ein röhrenförmiges Teil umfasst, welches eine offenzellige Schaumstruktur umfasst, wobei die Wärmequelle (12) mindestens teilweise innerhalb des röhrenförmigen Teils angeordnet ist, dadurch, dass die thermische Isolierung (18) ein röhrenförmiges Element umfasst, welches mindestens teilweise den Wärmeübertrager (13) umgibt und welches ein nanoporöses Material mit Poren von einer Größe kleiner als 50 nm umfasst, und dadurch, dass die Anordnung innerhalb eines Gehäuses (15) gelagert ist, welches zum Griff durch einen Benutzer vorgesehen ist.

2. Vaporisator nach Anspruch 1, **dadurch gekennzeichnet, dass** das nanoporöse Material mikroporös, mit Poren von einer Größe kleiner als 2 nm, ist.

3. Vaporisator nach einem der vorhergehenden Ansprüche, **dadurch gekennzeichnet, dass** die Isolierung (18) monolithische Strukturen aus Metalloxiden, amorphem Siliciumdioxid und Glasfilamenten umfasst.

4. Vaporisator nach einem der Ansprüche 1 bis 3, **dadurch gekennzeichnet, dass** die offenzellige Schaumstruktur eine spezifische Oberfläche von zwischen 50 und 200 cm²/cm³ hat.

5. Vaporisator nach einem der Ansprüche 1 bis 4, **dadurch gekennzeichnet, dass** die offenzellige Schaumstruktur metallisch ist.

6. Vaporisator nach einem der vorhergehenden Ansprüche, **dadurch gekennzeichnet, dass** er einen Kanal (14, 11, 17) für die Zirkulation des Luftstroms umfasst, wobei der Kanal eine Extraktionskammer (10) zur Unterbringung der stromabwärts von der Wärmequelle (12) bereitgestellten Substanz umfasst.

7. Vaporisator nach einem der vorhergehenden Ansprüche, **dadurch gekennzeichnet, dass** er weiterhin einen Luftbefeuchter (20) umfasst, der so bereitgestellt ist, dass er von dem Luftstrom durchflossen wird.

8. Vaporisator nach Anspruch 7, wenn er von Anspruch 6 abhängt, **dadurch gekennzeichnet, dass** der Luftbefeuchter (20) stromabwärts von der Extraktionskammer (10) bereitgestellt ist, so dass er von dem Luftstrom nach der Extraktion der Essenz durchflossen wird.

9. Vaporisator nach Anspruch 7, wenn er von Anspruch 6 abhängt, **dadurch gekennzeichnet, dass** der Luftbefeuchter stromaufwärts von der Extraktionskammer (10) bereitgestellt ist, so dass er von dem Luftstrom vor der Extraktion der Essenz durchflossen wird.

10. Vaporisator nach einem der vorhergehenden Ansprüche, **dadurch gekennzeichnet, dass** er eine Inhalationsdüse (60; 60'; 60") umfasst, durch die ein Benutzer Luft inhaliert, um den Luftstrom zu bewirken, der die Substanz durchfließt, aus der die Essenz zu extrahieren ist und die durch den Benutzer inhaliert wird.

11. Vaporisator nach einem der vorhergehenden Ansprüche, **dadurch gekennzeichnet, dass** er einen Dampfkollektor (5) zur Filtrierung des vom Benutzer nach der Extraktion der Essenz inhalierten Dampfes umfasst.

## Revendications

1. Vaporisateur d'essences portable pour l'extraction d'une essence et pour la fournir à un utilisateur, qui comprend une source de chaleur (12) pour échauffer un écoulement d'air qui est conduit à travers d'une substance de laquelle on peut extraire une essence, et qui est fourni à un utilisateur, comprenant en outre un échangeur de chaleur (13) fourni de façon que la chaleur générée par la source de chaleur (12) est fournie à l'écoulement d'air, et une isolation thermique (18) pour isoler la source de chaleur (12) et l'échangeur de chaleur (13) de l'extérieur, **caractérisé en ce que** l'échangeur de chaleur (13) comprend une pièce tubulaire qui comprend une structure de mousse à cellules ouvertes, étant la source de chaleur (12) fournie au moins en partie dans la pièce tubulaire, **en ce que** l'isolation thermique (18) comprend un élément tubulaire qui entoure au moins en partie l'échangeur de chaleur (13) et qui comprend un matériau nanoporeux avec des pores d'une taille inférieure à 50 nm, et **en ce que** l'ensemble est logé dans une boîte (15) qui est destinée à être saisie par un utilisateur.

2. Vaporisateur selon la revendication 1, **caractérisé en ce que** le matériau nanoporeux est microporeux, avec des pores d'une taille inférieure à 2 nm.

3. Vaporisateur selon l'une quelconque des revendications précédentes, **caractérisé en ce que** l'isolation (18) comprend des structures monolithiques d'oxydes de métal, de silice amorphe et de filaments de verre.

4. Vaporisateur selon l'une quelconque des revendications 1 à 3, **caractérisé en ce que** la structure de mousse à cellules ouvertes a une surface spécifique d'entre 50 et 200 cm²/cm³.

5. Vaporisateur selon l'une quelconque des revendications 1 à 4, **caractérisé en ce que** la structure de mousse à cellules ouvertes est métallique.

6. Vaporisateur selon l'une quelconque des revendications précédentes, **caractérisé en ce qu'**il comprend un conduit (14, 11, 17) pour la circulation de l'écoulement d'air, comprenant ledit conduit une chambre d'extraction (10) pour loger la substance étant fournie en aval de la source de chaleur (12).

7. Vaporisateur selon l'une quelconque des revendications précédentes, **caractérisé en ce qu'**il comprend en outre un humidificateur (20) fourni de façon qu'il est traversé par l'écoulement d'air.

8. Vaporisateur selon la revendication 7, lorsqu'elle dépend de la revendication 6, **caractérisé en ce que** l'humidificateur (20) est fourni en aval de la chambre d'extraction (10) de façon qu'il est traversé par l'écoulement d'air après l'extraction de l'essence.

9. Vaporisateur selon la revendication 7, lorsqu'elle dépend de la revendication 6, **caractérisé en ce que** l'humidificateur est fourni en amont de la chambre d'extraction (10), de façon qu'il est traversé par l'écoulement d'air avant de l'extraction de l'essence.

10. Vaporisateur selon l'une quelconque des revendications précédentes, **caractérisé en ce qu'**il comprend un bec d'inhalation (60 ; 60' ; 60") à travers lequel un utilisateur inhale de l'air afin de générer ledit écoulement d'air qui traverse la substance de laquelle on va extraire de l'essence et qui est inhalée par l'utilisateur.

11. Vaporisateur selon l'une quelconque des revendications précédentes, **caractérisé en ce qu'**il comprend un collecteur de vapeur (5) pour filtrer la vapeur exhalée par l'utilisateur après l'extraction de l'essence.
